# EUROPEAN PATENT APPLICATION

(11) **EP 2 031 074 A1**
(43) Date of publication of application: **04.03.2009**
(21) Application number: 07791494.3
(22) Date of filing: 27.07.2007
(51) Int. Cl.: C12Q 1/68, C12N 15/09, G01N 21/78, G01N 33/53, G01N 33/533

(54) **METHOD OF DETECTING VARIATION AND KIT TO BE USED THEREIN**

(30) Priority: 08.08.2006 JP 2006216194; 20.02.2007 JP 2007040078
(71) Applicant: Arkray, Inc., Kyoto-shi, Kyoto 601-8045 (JP)
(72) Inventor: MAJIMA, Satoshi, c/o Arkray, Inc., Kyoto-shi, Kyoto 601-8045 (JP); HIRAI, Mitsuharu, c/o Arkray, Inc., Kyoto-shi, Kyoto 601-8045 (JP); MAEKAWA, Taira, c/o Arkray, Inc., Kyoto-shi, Kyoto 601-8045 (JP); KIMURA, Shinya, c/o Arkray, Inc., Kyoto-shi, Kyoto 601-8045 (JP)
(74) Representative: Behnisch, Werner
(86) International application number: PCT/JP2007/064800
(87) International publication number: WO 2008/018305

(57) **Abstract**

A method of detecting a mutation is provided that uses Tm analysis and is excellent in detection sensitivity. A detection probe consisting of a polynucleotide complementary to a sequence to be detected containing a detection site that has been mutated and an inhibitory polynucleotide complementary to a sequence not to be detected containing the detection site that is unmutated are added to a sample containing a DNA to be detected in which the detection site has been mutated and a DNA not to be detected in which the detection site is unmutated, so that the detection probe is hybridized with the DNA. Then while the hybridization product between the DNA and the detection probe is heated, a signal variation associated with an increase in temperature is measured, then the signal variation is analyzed, and thereby a Tm value is determined, based on which the presence of the mutation is determined.

[Sequence Table] TF07027-01.ST25.txt

SEQUENCE LISTING

<110> ARKRAY, Inc.

<120> Method for detection of mutation and kit therefor

<130> TF07027-01

<150> JP2006/216194
<151> 2006-08-08

<150> JP2007/040078
<151> 2007-02-20

<160> 16

<170> PatentIn version 3.1

<210> 1
<211> 5381
<212> DNA
<213> Homo sapiens

<400> 1
   atgttggaga tctgcctgaa gctggtgggc tgcaaatcca agaaggggct gtcctcgtcc 60
   tccagctgtt atctggaaga agcccttcag cggccagtag catctgactt tgagcctcag 120
   ggtctgagtg aagccgctcg ttggaactcc aaggaaaacc ttctcgctgg acccagtgaa 180
   aatgacccca accttttcgt tgcactgtat gattttgtgg ccagtggaga taacactcta 240
   agcataacta aaggtgaaaa gctccgggtc ttaggctata atcacaatgg ggaatggtgt 300
   gaagcccaaa ccaaaaatgg ccaaggctgg gtcccaagca actacatcac gccagtcaac 360
   agtctggaga aacactcctg gtaccatggg cctgtgtccc gcaatgccgc tgagtatctg 420
   ctgagcagcg ggatcaatgg cagcttcttg gtgcgtgaga gtgagagcag tcctggccag 480
   aggtccatct cgctgagata cgaagggagg gtgtaccatt acaggatcaa cactgcttct 540
   gatggcaagc tctacgtctc ctccgagagc cgcttcaaca ccctggccga gttggttcat 600
   catcattcaa cggtggccga cgggctcatc accacgctcc attatccagc cccaaagcgc 660
   aacaagccca ctgtctatgg tgtgtccccc aactacgaca agtgggagat ggaacgcacg 720
   gacatcacca tgaagcacaa gctgggcggg ggccagtacg gggaggtgta cgagggcgtg 780
   tggaagaaat acagcctgac ggtggccgtg aagaccttga aggaggacac catggaggtg 840
   gaagagttct tgaaagaagc tgcagtcatg aaagagatca aacaccctaa cctggtgcag 900
   ctccttgggg tctgcacccg ggagcccccg ttctatatca tcactgagtt catgacctac 960
   gggaacctcc tggactacct gagggagtgc aaccggcagg aggtgaacgc cgtggtgctg 1020
   ctgtacatgg ccactcagat ctcgtcagcc atggagtacc tggagaagaa aaacttcatc 1080
   cacagagatc ttgctgcccg aaactgcctg gtaggggaga accacttggt gaaggtagct 1140
   gattttggcc tgagcaggtt gatgacaggg gacacctaca cagcccatgc tggagccaag 1200
   ttccccatca aatggactgc acccgagagc ctggcctaca acaagttctc catcaagtcc 1260
   gacgtctggg catttggagt attgctttgg gaaattgcta cctatggcat gtccccttac 1320
   ccgggaattg acctgtccca ggtgtatgag ctgctagaga aggactaccg catggagcgc 1380
   ccagaaggct gcccagagaa ggtctatgaa ctcatgcgag catgttggca gtggaatccc 1440
   tctgaccggc cctcctttgc tgaaatccac caagcctttg aaacaatgtt ccaggaatcc 1500
   agtatctcag acgaagtgga aaaggagctg gggaaacaag gcgtccgtgg ggctgtgagt 1560
   accttgctgc aggccccaga gctgcccacc aagacgagga cctccaggag agctgcagag 1620
   cacagagaca ccactgacgt gcctgagatg cctcactcca agggccaggg agagagcgat 1680
   cctctggacc atgagcctgc cgtgtctcca ttgctccctc gaaaagagcg aggtcccccg 1740
   gagggcggcc tgaatgaaga tgagcgcctt ctccccaaag acaaaaagac caacttgttc 1800
   agcgccttga tcaagaagaa gaagaagaca gccccaaccc ctcccaaacg cagcagctcc 1860
   ttccgggaga tggacggcca gccggagcgc agaggggccg gcgaggaaga gggccgagac 1920
   atcagcaacg gggcactggc tttcaccccc ttggacacag ctgacccagc caagtcccca 1980
   aagcccagca atggggctgg ggtccccaat ggagccctcc gggagtccgg gggctcaggc 2040
   ttccggtctc cccacctgtg gaagaagtcc agcacgctga ccagcagccg cctagccacc 2100
   ggcgaggagg agggcggtgg cagctccagc aagcgcttcc tgcgctcttg ctccgcctcc 2160
   tgcgttcccc atggggccaa ggacacggag tggaggtcag tcacgctgcc tcgggacttg 2220
   cagtccacgg gaagacagtt tgactcgtcc acatttggag ggcacaaaag tgagaagccg 2280
   gctctgcctc ggaagagggc aggggagaac aggtctgacc aggtgacccg aggcacagta 2340
   acgcctcccc ccaggctggt gaaaaagaat gaggaagctg ctgatgaggt cttcaaagac 2400
   atcatggagt ccagcccggg ctccagcccg cccaacctga ctccaaaacc cctccggcgg 2460
   caggtcaccg tggcccctgc ctcgggcctc ccccacaagg aagaagctgg aaagggcagt 2520
   gccttaggga cccctgctgc agctgagcca gtgaccccca ccagcaaagc aggctcaggt 2580
   gcaccagggg gcaccagcaa gggccccgcc gaggagtcca gagtgaggag gcacaagcac 2640
   tcctctgagt cgccagggag ggacaagggg aaattgtcca ggctcaaacc tgccccgccg 2700
   cccccaccag cagcctctgc agggaaggct ggaggaaagc cctcgcagag cccgagccag 2760
   gaggcggccg gggaggcagt cctgggcgca aagacaaaag ccacgagtct ggttgatgct 2820
   gtgaacagtg acgctgccaa gcccagccag ccgggagagg gcctcaaaaa gcccgtgctc 2880
   ccggccactc caaagccaca gtccgccaag ccgtcgggga cccccatcag cccagccccc 2940
   gttccctcca cgttgccatc agcatcctcg gccctggcag gggaccagcc gtcttccacc 3000
   gccttcatcc ctctcatatc aacccgagtg tctcttcgga aaacccgcca gcctccagag 3060
   cggatcgcca gcggcgccat caccaagggc gtggtcctgg acagcaccga ggcgctgtgc 3120
   ctcgccatct ctaggaactc cgagcagatg gccagccaca gcgcagtgct ggaggccggc 3180
   aaaaacctct acacgttctg cgtgagctat gtggattcca tccagcaaat gaggaacaag 3240
   tttgccttcc gagaggccat caacaaactg gagaataatc tccgggagct tcagatctgc 3300
   ccggcgacag caggcagtgg tccagcggcc actcaggact tcagcaagct cctcagttcg 3360
   gtgaaggaaa tcagtgacat agtgcagagg tagcagcagt caggggtcag gtgtcaggcc 3420
   cgtcggagct gcctgcagca catgcgggct cgcccatacc cgtgacagtg gctgacaagg 3480
   gactagtgag tcagcacctt ggcccaggag ctctgcgcca ggcagagctg agggccctgt 3540
   ggagtccagc tctactacct acgtttgcac cgcctgccct cccgcacctt cctcctcccc 3600
   gctccgtctc tgtcctcgaa ttttatctgt ggagttcctg ctccgtggac tgcagtcggc 3660
   atgccaggac ccgccagccc cgctcccacc tagtgcccca gactgagctc tccaggccag 3720
   gtgggaacgg ctgatgtgga ctgtcttttt catttttttc tctctggagc ccctcctccc 3780
   ccggctgggc ctccttcttc cacttctcca agaatggaag cctgaactga ggccttgtgt 3840
   gtcaggccct ctgcctgcac tccctggcct tgcccgtcgt gtgctgaaga catgtttcaa 3900
   gaaccgcatt tcgggaaggg catgcacggg catgcacacg gctggtcact ctgccctctg 3960
   ctgctgcccg gggtggggtg cactcgccat ttcctcacgt gcaggacagc tcttgatttg 4020
   ggtggaaaac agggtgctaa agccaaccag cctttgggtc ctgggcaggt gggagctgaa 4080
   aaggatcgag gcatggggca tgtcctttcc atctgtccac atccccagag cccagctctt 4140
   gctctcttgt gacgtgcact gtgaatcctg gcaagaaagc ttgagtctca agggtggcag 4200
   gtcactgtca ctgccgacat ccctccccca gcagaatgga ggcaggggac aagggaggca 4260
   gtggctagtg gggtgaacag ctggtgccaa atagccccag actgggccca ggcaggtctg 4320
   caagggccca gagtgaaccg tcctttcaca catctgggtg ccctgaaagg gcccttcccc 4380
   tcccccactc ctctaagaca aagtagattc ttacaaggcc ctttcctttg gaacaagaca 4440
   gccttcactt ttctgagttc ttgaagcatt tcaaagccct gcctctgtgt agccgccctg 4500
   agagagaata gagctgccac tgggcacctg cgcacaggtg ggaggaaagg gcctggccag 4560
   tcctggtcct ggctgcactc ttgaactggg cgaatgtctt atttaattac cgtgagtgac 4620
   atagcctcat gttctgtggg ggtcatcagg gagggttagg aaaaccacaa acggagcccc 4680
   tgaaagcctc acgtatttca cagagcacgc ctgccatctt ctccccgagg ctgccccagg 4740
   ccggagccca gatacggggg ctgtgactct gggcagggac ccggggtctc ctggaccttg 4800
   acagagcagc taactccgag agcagtgggc aggtggccgc ccctgaggct tcacgccggg 4860
   agaagccacc ttcccacccc ttcataccgc ctcgtgccag cagcctcgca caggccctag 4920
   ctttacgctc atcacctaaa cttgtacttt atttttctga tagaaatggt ttcctctgga 4980
   tcgttttatg cggttcttac agcacatcac ctctttgccc ccgacggctg tgacgcagcc 5040
   ggagggaggc actagtcacc gacagcggcc ttgaagacag agcaaagcgc ccacccaggt 5100
   cccccgactg cctgtctcca tgaggtactg gtcccttcct tttgttaacg tgatgtgcca 5160
   ctatatttta cacgtatctc ttggtatgca tcttttatag acgctctttt ctaagtggcg 5220
   tgtgcatagc gtcctgccct gccccctcgg gggcctgtgg tggctccccc tctgcttctc 5280
   ggggtccagt gcattttgtt tctgtatatg attctctgtg gttttttttg aatccaaatc 5340
   tgtcctctgt agtatttttt aaataaatca gtgtttacat t 5381

<210> 2
<211> 16
<212> DNA
<213> Artificial

<220>
<223> probe

<400> 2
ccgtagtggc ccccgc 16

<210> 3
<211> 16
<212> DNA
<213> Artificial

<220>
<223> polynucleotide

<400>  3
ccgtactggc ccccgc                                                        16


<210>  4
<211>  16
<212>  DNA
<213>  Artificial

<220>
<223>  probe

<400>  4
ccgaactggc ccccgc                                                        16


<210>  5
<211>  20
<212>  DNA
<213>  Artificial

<220>
<223>  polynucleotide

<400>  5
   cctccccgta ctggcccccg                                                   20


<210>  6
<211>  16
<212>  DNA
<213>  Artificial

<220>
<223>  polynucleotide

<400>  6
ccgtactggc ccccgc                                                        16


<210>  7
<211>  17
<212>  DNA
<213>  Artificial

<220>
<223>  purobe

<400>  7
ccagtacggg aaggtgt                                                       17


<210>  8
<211>  17
<212>  DNA
<213>  Artificial

<220>
<223>  polynucleotide

<400>  8
ccagtacggg gaggtgt                                                       17


<210>  9
<211>  19
<212>  DNA
<213>  Artificial

<220>
<223>  sense primer

<400>  9
ggagatggaa cgcacggac                                                     19

<210>  10
<211>  16
<212>  DNA
<213>  Artificial

<220>
<223>  antisense primer

<400>  10
ggccaccgtc aggctg                                                        16


<210>  11
<211>  21
<212>  DNA
<213>  Artificial

<220>
<223>  sense primer

<400>  11
gacaagtggg agatggaacg c                                                  21


<210>  12
<211>  16
<212>  DNA
<213>  Artificial

<220>
<223>  antisense primer

<400>  12
cacggccacc gtcagg                                                        16


<210>  13
<211>  21
<212>  DNA
<213>  Artificial

<220>
<223>  sense primer

<400>  13
gacaagtggg agatggaacg c                                                  21


<210>  14
<211>  16
<212>  DNA
<213>  Artificial

<220>
<223>  antisense primer

<400>  14
cacggccacc gtcagg                                                        16


<210>  15
<211>  21
<212>  DNA
<213>  Artificial

<220>
<223>  sense primer

<400>  15
gacaagtggg agatggaacg c                                                  21


<210>  16
<211>  16

<212>  DNA
<213>  Artificial

<220>
<223>  antisense primer

<400>  16
cacggccacc gtcagg                                                    16

## Description

### Technical Field

The present invention relates generally to a method of detecting a mutation and a kit used in the same.

### Background Art

Detection of point mutation, so-called single nucleotide polymorphism (SNP), is being employed widely as a method of analyzing, at the gene level, for example, the causes of all types of diseases and individual differences in disease liability (susceptibility to diseases) and in drug action (drug efficacy).

Examples of common methods of detecting a point mutation include (1) a direct sequencing method where the region corresponding to the sequence to be detected in the target DNA of a sample is amplified and the base sequence of the resultant amplification product is analyzed, (2) a pyrosequencing method, (3) a denaturing HPLC method where the region corresponding to the sequence to be detected is amplified, the resultant amplification product is subjected to HPLC in a temperature gradient column, and the presence of any mutation is detected according to the time that is required for elution, (4) an invadar method where a mutation is detected through the detection of fluorescence that is emitted when a fluorescent probe binds to the region containing the target mutation, and (5) the ASP-PCR method where PCR is performed using a primer with the target mutation located at the 3'-terminal region and the mutation is judged based on whether or not amplification occurs.

However, the aforementioned methods (1), (2), and (4) are not very sensitive, specifically their sensitivity is about 20%, about 5%, and about 5%, respectively, and require a considerable amount of time and labor for their operations. The aforementioned method (3) has problems in that the sensitivity is as low as about 10%, it only can check whether or not any mutation has occurred, it cannot analyze the site and type of a mutation, and it lacks specificity. The aforementioned method (5) is highly sensitive but is less specific, so that it is apt to give a false-positive result, which is a problem. In this context, the lower the value (%) is, the higher the sensitivity.

Because of these problems, recently, a detection method using Tm analysis has been employed for detection of point mutations. This method can be performed, for example, as follows. First, a probe complementary to the sequence to be detected containing a point mutation to be detected is used to form a hybrid (double-stranded DNA) between the target single-stranded DNA in the sample and the aforementioned probe. Subsequently, this hybridization product is heat-treated so that dissociation (melting) of the hybrid with an increase in temperature is detected by measurement of a signal such as absorbance. The presence or absence of any point mutation is judged based on the Tm value determined through the detection. The higher the Tm value, the higher the homology of the hybridization product, and the lower the Tm value, the lower the homology. Therefore the judgment described below can be made when the Tm value (reference value for assessment) is determined beforehand for the hybridization product between the sequence to be detected containing a point mutation and a probe complementary thereto and then the Tm value (measured value) of the hybridization product between the target single-stranded DNA and the aforementioned probe is measured. When the aforementioned measured value is identical to the aforementioned reference value, it is considered as matching, that is it can be judged that the point mutation is present in the target DNA. On the other hand, when the aforementioned measured value is lower than the aforementioned reference value, it is considered as mismatching, that is, it can be judged that no point mutation is present in the target DNA.

However, the detection method using such Tm analysis has a problem in that the sensitivity is low. In a specific example, the problem arises in detecting a point mutation in DNA derived from blood cells of leukemia patients (Patent Document 1). Leukemia is a disease resulting from malignant transformation of hematopoietic stem cells in the bone marrow. Chronic myeloid leukemia (CML), among others, is known to have its origin in the bcr-abl fusion gene generated by translocation between chromosome 9 and chromosome 22, and for example, imatinib, an ABL kinase inhibitor, is used widely for the treatment of the disease. However, when a point mutation is present in the abl gene (including the abl gene in the aforementioned fusion gene), resistance to imatinib is developed, which is a problem. In that case, for example, an increase in dose of imatinib, a change to any other therapeutic drug, or switching to, for example, bone marrow transplantation becomes necessary for the treatment. Therefore in the treatment of leukemia, particularly CML, it is very important to detect the presence of a point mutation in the abl gene. However, even in the blood of the same CML patient, there are blood cells with point mutations (the sequences to be detected) and those without point mutations (sequences not to be detected) and the difference between these two types of blood cells resides merely in a point mutation, that is, a single base of the sequence. Then a phenomenon may occur where the probe to detect a point mutation is hybridized not only with the sequence to be detected containing the point mutation (match) but also with a sequence not to be detected that does not contain the point mutation (mismatch). In this case, when a melting curve that shows the relationship between signal intensity and temperature is prepared based on Tm analysis, it is difficult to detect the peak on the higher temperature side corresponding to the matched sequence to be detected due to the presence of the peak on the lower temperature side corresponding to the mismatched sequence not to be detected, which further reduces the detection sensitivity.
[Patent Document 1] JP 2004-537992 A

### Disclosure of Invention

### Problem to be Solved by the Invention

Hence, an object of the present invention is to provide a method of detecting a mutation that uses Tm analysis and is excellent in detection sensitivity, and a detection probe kit used in the method.

### Means for Solving the Problem

In order to achieve the aforementioned object, the method of detecting a mutation of the present invention is characterized in that a sample contains a DNA to be detected in which a detection site has been mutated and a DNA not to be detected in which the detection site is unmutated, and
the method includes the following steps (A) to (E):
(A) a step of adding a detection probe consisting of a polynucleotide complementary to a sequence to be detected and a polynucleotide complementary to a sequence not to be detected (hereinafter, referred to as 'inhibitory polynucleotide') to the sample containing the DNA,
   wherein the sequence to be detected is the DNA to be detected or a partial sequence thereof and contains the detection site that has been mutated, and
   the sequence not to be detected is the DNA not to be detected or a partial sequence thereof and contains the detection site that is unmutated,
(B) a step of hybridizing the detection probe to the DNA,
(C) a step of measuring a signal variation associated with a change in temperature with respect to a hybridization product between the DNA and the detection probe,
(D) a step of determining a Tm value based on analysis of the signal variation, and
(E) a step of determining the presence or absence of a mutation at the site to be detected, based on the Tm value.

The detection probe kit of the present invention is a detection probe kit used in the method of detecting a mutation of the present invention,
wherein it includes a detection probe consisting of a polynucleotide complementary to a sequence to be detected and an inhibitory polynucleotide complementary to a sequence not to be detected,
the sequence to be detected is a DNA to be detected in which the detection site has been mutated or a partial sequence thereof and contains the detection site that has been mutated, and
the sequence not to be detected is the DNA not to be detected in which the detection site is unmutated or a partial sequence thereof and contains the detection site that is unmutated.

### Effect of the Invention

In the method of detecting a mutation of the present invention, not only the detection probe for the sequence to be detected in which the detection site has been mutated but also an inhibitory polynucleotide for the sequence not to be detected in which the detection site is unmutated are added to the sample. As a result, hybridization of the detection probe to the sequence not to be detected can be suppressed, and as a result, a mutation located at the detection site can be detected at a higher sensitivity (about 3%) as compared with the conventional method. Accordingly, the suppression of hybridization of the detection probe to the sequence not to be detected results from the higher homology of the inhibitory polynucleotide to the sequence not to be detected, as compared to the detection probe. Therefore the detection method of the present invention is useful for, for example, a sample containing both a DNA to be detected and a DNA not to be detected. The method is useful particularly for samples from leukemia patients, and especially useful in detecting a mutation of an abl gene (including a mutation of the abl gene in a bcr-abl fusion gene) in patients with chronic myeloid leukemia (CML). As described above, since a mutation can be detected at a high sensitivity, for example, it is possible to analyze at the gene level the adequacy of a therapeutic drug for leukemia for individual patients, and therefore the method of the present invention is considered to be very useful in the field of medical care. The method of detecting a mutation of the present invention can be carried out simply and easily by using the detection probe kit of the present invention.

### Brief Description of Drawings

FIG. 1 shows graphs indicating the results of Tm analysis with an inhibitory polynucleotide added in Example 1 of the present invention.
FIG. 2 shows graphs indicating the results of Tm analysis with no inhibitory polynucleotide added in Comparative Example 1.
FIG. 3 shows graphs indicating the results of Tm analysis in which the ratio of the detection probe to be added was varied, in Example 1 of the present invention.
FIG. 4 shows graphs indicating the results of Tm analysis in which the ratio of the inhibitory polynucleotide to be added was varied, in Example 1 of the present invention.
FIG. 5 shows graphs indicating the results of Tm analysis with an inhibitory polynucleotide added in Example 2 of the present invention.
FIG. 6 shows graphs indicating the results of Tm analysis with an inhibitory polynucleotide added in Example 3 of the present invention.
FIG. 7 shows graphs indicating the results of Tm analysis with an inhibitory polynucleotide added in Example 4 of the present invention.

### Best Mode for Carrying Out the Invention

As described above, the method of detecting a mutation of the present invention is a method of detecting a mutation of a DNA contained in a sample and is characterized in that the aforementioned sample contains a DNA to be detected in which a detection site has been mutated and a DNA not to be detected in which the detection site is unmutated, and the method includes the following steps (A) to (E):
(A) a step of adding a detection probe consisting of a polynucleotide complementary to a sequence to be detected and an inhibitory polynucleotide complementary to a sequence not to be detected, to the sample containing the DNA,
(B) a step of hybridizing the detection probe to the DNA,
(C) a step of measuring a signal variation associated with a change in temperature with respect to a hybridization product between the DNA and the detection probe,
(D) a step of determining a Tm value based on analysis of the signal variation, and
(E) a step of determining the presence or absence of a mutation at the site to be detected, based on the Tm value.

In the step (A), the aforementioned sequence to be detected is the DNA to be detected or a partial sequence thereof and contains the detection site that has been mutated. The aforementioned sequence not to be detected is the DNA not to be detected or a partial sequence thereof and contains the detection site that is unmutated.

In the present invention, the aforementioned sequence to be detected in which a mutation is present at the detection site also may be referred to as a 'mutated sequence', the DNA to be detected that contains the sequence to be detected also may be referred to as a 'mutated DNA', the aforementioned sequence not to be detected in which no mutation is present at the detection site also may be referred to as a 'normal sequence', and the DNA containing the aforementioned sequence not to be detected also may be referred to as a 'normal DNA'. The mutation located at the detection site also may be referred to as a 'detection target mutation'. The DNA contained in the sample that is the target for detection of the presence or absence of a mutation also may be referred to as a 'target DNA'. The mutation to be detected in the present invention is, for example, single nucleotide polymorphism (SNP).

In the present invention, the DNA contained in the sample may be a single-stranded DNA or a double-stranded DNA. When the DNA is a double-stranded DNA, it is preferable that, for example, a step of dissociating the double-stranded DNA contained in the sample by heating before the hybridizing step (B) be included. Dissociation of a double-stranded DNA into single-stranded DNAs allows efficient hybridization with the detection probe or the inhibitory polynucleotide in the subsequent hybridizing step (B).

In the present invention, a DNA contained in the sample may be, for example, a gene or a partial sequence of a gene. A DNA contained in the aforementioned sample may be, for example, a DNA intrinsically contained in the sample such as a biological sample. However, it is preferably an amplification product obtained through amplification carried out by a gene amplification method since this can improve the detection accuracy. Specific examples include an amplification product obtained through amplification carried out by the gene amplification method using, as a template, DNA intrinsically contained in the sample, and an amplification product obtained through amplification carried out by the gene amplification method using, as a template, cDNAproduced through a reverse transcription reaction (for example, RT-PCR (reverse transcription PCR)) from an RNA (for instance, a total RNA and an mRNA) intrinsically contained in the sample. The length of the amplification product is not particularly limited and is, for example, 50 to 1000 mer, preferably 80 to 200 mer.

The sample to which the detection method of the present invention is applied is not particularly limited. The present invention is, as described above, very useful for, for example, a sample that contains both DNA with a target mutation (DNA to be detected) as the target DNA and DNA with no target mutation (DNA not to be detected). The source of the aforementioned DNA and RNA is not limited. Examples thereof include cells such as various cancer cells, virus, and mitochondria. As described above, when the detection of a mutation is carried out with respect to a biological sample (for example, blood sample) obtained from a leukemia patient, the aforementioned problem tends to occur because malignantly transformed blood cells contain cells with mutated DNAs and cells with non-mutated DNAs. Therefore it is preferable that the detection method of the present invention be used particularly for a sample containing a mutated DNA and a non-mutated DNA, for example, a biological sample obtained from a leukemia patient, specifically, for instance, a blood sample and leukocyte cells. In the present invention, the method of collecting the sample, the method of preparing a DNA, etc. are not limited and conventionally known methods can be employed.

The detection target mutation in the present invention is not limited. As described above, it is known that the detection probe hybridizes with a sequence not to be detected, in detecting a leukemia-related mutation. This indicates that the method of the present invention is useful in detecting a mutation in a leukemia-related gene as a specific example. The mutation in a leukemia-related gene is, for example, a mutation of an abl gene (including a mutation of the abl gene in a bcr-abl fusion gene). Examples of the mutation include those of the 756th base G, the 758th base A, and the 763rd base G in the cDNA sequence (mRNA sequence) of the abl gene indicated in Sequence No. 1. With respect to these bases, for example, mutations to the following bases have been reported. The sequence of the abl gene has been deposited at NCBI accession No. NM_005157.
Mutation G756C Mutation of 756th base G to C
Mutation A758T Mutation of 758th base A to T
Mutation G763A Mutation of 763rd base G to A

In the present invention, the aforementioned detection probe may be any sequence as long as it is complementary to the aforementioned sequence to be detected in which the detection site has been mutated, and the aforementioned inhibitory polynucleotide may be of any sequence as long as it is complementary to the aforementioned sequence not to be detected in which the detection site is unmutated. The length of the probe and that of the inhibitory polynucleotide are not particularly limited but are preferably the same. The sequence of the detection probe and that of the inhibitory polynucleotide are preferably identical to each other within 90 to 100%, particularly preferably 100% except the site (base) to be paired with the detection site (the site where the target mutation occurs) during hybridization. The detection probe and the inhibitory polynucleotide may be designed so that, for example, they may be hybridized either to the forward strand or the reverse strand of DNA as long as they hybridize to the same strand.

Examples of the combination between a detection probe and an inhibitory polynucleotide that are used for detection of the aforementioned three types of mutations (G756C, A758T, G763A) of the abl gene are indicated below. In each sequence, the bases indicated in capital letters represent the 756th, 758th, and 763rd bases of the abl gene, respectively. The invention is not limited to these combinations.

### Mutation G756C (for detection in forward strand)

Detection probe Sequence No. 2
5'-ccgtaGtggcccccgc-3'

Inhibitory polynucleotide Sequence No. 3
5'-ccgtaCtggcccccgc-3'

### Mutation A758T (for detection in forward strand)

Detection probe Sequence No. 4
5'-ccgAactggcccccgc' 3'

Inhibitory polynucleotide Sequence No. 5
5'-cctccccgTactggcocceg-3'

Inhibitory polynucleotide Sequence No. 6
5'-ccgTactggcccccgc- 3'

### Mutation G763A (for detection in reverse strand)

Detection probe Sequence No. 7
5'-ccagtacgggAaggtgt-3'

Inhibitory polynucleotide Sequence No. 8
5'-ccagtacgggGaggtgt-3'

In the present invention, the ratio of the inhibitory polynucleotide to be added is not particularly limited and can be determined suitably according to the conditions for the detection system, for example, the length of the detection probe and the GC content in the sequence to be detected. The ratio of that to be added to the detection probe is not particularly limited, but the lower limit thereof is, for example, 0.1 times or more, preferably equal or more, and more preferably twice or more in molar ratio. On the other hand, the upper limit is, for example, 100 times or less in molar ratio. The length of the aforementioned inhibitory polynucleotide is not particularly limited and is, for example, 5 to 50 mer, preferably 10 to 30 mer, and is preferably the same as that of the detection probe.

In the present invention, the ratio of the detection probe to be added that is complementary to the sequence to be detected is not particularly limited. However, it is preferably equal or less, in molar ratio, to DNA in the sample because these ratios allow detection signals to be obtained satisfactorily, for example. Not only addition of the inhibitory polynucleotide but also control of the ratio of the detection probe to be added can increase further, for example, the detection sensitivity. Furthermore, operation is very simple because it is only necessary to set the ratio of the detection probe to be added. The ratio of the detection probe to be added is more preferably 0.1 times or less in molar ratio to the DNA. In this context, the DNA contained in the sample may be the total of DNA to be detected and DNA not to be detected or the total of amplification products containing the sequence to be detected and amplification products containing the sequence not to be detected. The ratio of the DNA to be detected to the DNA contained in the sample is generally unknown. However, in the end, the ratio of the detection probe to be added is, for example, preferably 10 times or less, more preferably 5 times or less, and further preferably 3 times or less in molar ratio to the DNA to be detected (or the amplification product containing the sequence to be detected). The lower limit thereof is not particularly limited. For example, it is preferably 0.001 times or more, more preferably 0.01 times or more, and further preferably 0.1 times or more in molar ratio to the DNA to be detected.

The ratio of the detection probe to be added to the DNA may be expressed, for example, in molar ratio to the double-stranded DNA or in molar ratio to the single-stranded DNA. The length of the detection probe is not particularly limited and is, for example, 5 to 50 mer, preferably 10 to 30 mer.

The Tm value is described below. When a solution containing a double-stranded DNA is heated, absorbance at 260 nm is increased. This is caused as follows, that is, heating breaks the hydrogen bonds formed between the strands of the double-stranded DNA to dissociate the double-stranded DNA into single-stranded DNAs (melting of DNA). When all double-stranded DNAs are dissociated into single-stranded DNAs, the absorbance thereof becomes about 1.5 times the absorbance obtained at the start of heating (the absorbance of double-stranded DNAs alone), based on which it can be judged that melting has completed. Based on this phenomenon, the melting temperature Tm is defined as the temperature at which the absorbance reaches 50% of the full increase of the absorbance.

In the present invention, measurement of signal variations associated with an increase in temperature caused for determination of the Tm value can be made by, for example, measurement of absorbance at 260 nm based on the aforementioned principle. More preferably, signal variations are measured using a probe labeled with a labeling substance as the aforementioned detection probe. The labeled site in the detection probe is not particularly limited. Furthermore, the labeling substance is not particularly limited and usually it can be bound to the phosphate group in a nucleotide.

The aforementioned labeled probe is, for example, a labeled probe that shows a signal by itself but shows no signal after hybridization or a labeled probe that shows no signal by itself but shows a signal after hybridization. The former probe shows no signal when kept hybridized with the sequence to be detected (double-stranded DNA) and shows a signal when the probe has been released by heating. The latter probe shows a signal when kept hybridized with the sequence to be detected (double-stranded DNA) and the signal is reduced (disappears) when the probe has been released by heating. Therefore, for example, the signal generated by the labeling substance is detected under the conditions (for example, absorbance) peculiar to the signal, and thereby the progress of melting and the Tm value can be determined, as in the case of the measurement of absorbance at 260 nm.

The labeling substance is not limited and is, for example, a fluorochrome (fluorophore). A preferred specific example of the aforementioned labeled probe is a probe that is labeled with a fluorochrome and that emits fluorescence by itself and emits weakened (for instance, quenched) fluorescence after hybridization. Probes making use of such a fluorescence quenching phenomenon are called fluorescence quenching probes. Among these probes, a preferable detection probe has the 3'-terminal or 5'-terminal of oligonucleotide labeled with a fluorochrome, and the base of the aforementioned terminal to be labeled is preferably C. In this case, it is preferable that the base sequence of the detection probe be designed so that in the DNA to be detected, with which the detection probe hybridizes, the base to be paired with the terminal base C of the detection probe or the base that is spaced 1 to 3 bases from the aforementioned base to be paired is G. Generally, such a probe is called a guanine quenching probe and is known as the so-called QProbe (registered trademark). When such a guanine quenching probe hybridizes with a DNA to be detected, the terminal base C labeled with a fluorochrome approaches G of the DNA to be detected, so that the phenomenon that emission of the fluorochrome is weakened (fluorescence intensity is decreased) occurs.

The fluorochrome is not particularly limited and is, for example, a fluorescein, a phosphor, a rhodamine, or a polymethine dye derivative. Examples of commercially available fluorochromes include BODIPY FL (trade mark, manufactured by Molecular Probes), FluorePrime (trade name, manufactured by Amersham Pharmacia), Fluoredite (trade name, manufactured by Millipore), FAM (manufactured by ABI), Cy3 and Cy5 (manufactured by Amersham Pharmacia), and TARMA (manufactured by Molecular Probes). Detection conditions are not particularly limited and can be decided suitably according to the fluorochrome to be used. Specifically, for example, Pacific Blue can be detected at, for example, a detection wavelength of 450 to 480 nm, TAMRA at, for example, a detection wavelength of 585 to 700 nm, and BODIPY FL at, for example, a detection wavelength of 515 to 555 nm. With such a probe, hybridization and dissociation can be confirmed easily based on signal variations. On the other hand, it is preferable that the inhibitory polynucleotide be unlabeled.

In the following, the detection method of the present invention is described using, as an example, the point mutation (A to T) at the 758th base A in the abl gene (Sequence No. 1). The present invention is characterized in that an inhibitory polynucleotide is added, and other steps and conditions are not limited. The sequence to be detected, with which the detection probe hybridizes, may be, for example, the full-length sequence with the 758th base A mutated to T in the base sequence of Sequence No. 1, but preferably a partial sequence as long as it contains the 758th base (A to T), the detection site.

First, a genomic DNA is isolated from whole blood. Isolation of a genomic DNA from whole blood can be performed by a conventionally known method and, for example, a commercially available genomic DNA isolation kit (trade name: GFX Genomic Blood DNA Purification kit; manufactured by GE Healthcare BioScience) can be used.

Next, a detection probe and an inhibitory polynucleotide are added to a sample containing the isolated genomic DNA. The timing of addition of the detection probe and the inhibitory polynucleotide is not limited as described below. In the present embodiment, a method where the inhibitory polynucleotide is added after addition of the detection probe is described as an example.

That is, a detection probe is added to a sample containing the isolated genomic DNA first. Examples of the aforementioned detection probe include those described above, among which QProbe is preferable. The QProbe whose base terminal is, in general, cytosine as described above is a probe with the terminal labeled with a fluorochrome. Hybridization of the probe with the sequence to be detected causes an interaction between the fluorochrome and guanine in the sequence to be detected, which results in weakened (or quenched) fluorescence.

The sequence of the detection probe is not limited as long as it is complementary to the sequence to be detected containing a point mutation, and can be designed suitably according to the sequence to be detected. When the point mutation (A to T) at the 758th base A in the abl gene (Sequence No. 1) is to be detected, for example, a polynucleotide consisting of the aforementioned base sequence of the Sequence No. 4 is used.

Detection probe for mutation A758T Sequence No. 4
5'-ccgaActggcccccgc-3' (GC content: 81.3%)

Similarly in the case where a gene amplification method is performed using a DNA or an RNA contained in the sample as a template, timing of addition of the detection probe is not particularly limited. For example, the detection probe may be added to the resultant amplification product after the gene amplification treatment described below. However, it is preferable that the detection probe be added before the gene amplification treatment. In the case where the detection probe is added before the gene amplification treatment as described above, in order to prevent the probe itself from elongating, for example, another phosphate group may be added to the 3'-terminal thereof or the 3'-terminal may be labeled with a fluorochrome as described above.

The detection probe may be added to, for example, a liquid sample containing the isolated genomic DNA or may be mixed with the genomic DNA in a solvent. The solvent is not particularly limited. Examples thereof include conventionally known solvents, for example, a buffer solution such as Tris-HCl, a solvent containing KCl, MgCl₂, MgSO₄, or glycerol, and a PCR reaction solution.

Subsequently, amplification of the target sequence is performed by the gene amplification method using the isolated genomic DNA as a template. Specifically, the sequence containing the base site where the point mutation to be detected occurs, that is, the sequence to be detected and the sequence not to be detected are amplified.

The gene amplification method is not limited and examples thereof include the PCR (polymerase chain reaction) method, NASBA (nucleic acid sequence based amplification) method, TMA (transcription-mediated amplification) method, and SDA (strand displacement amplification) method, among which the PCR method is preferable. In the following, the present invention is described using the PCR method as an example but is not limited thereto. The conditions for PCR are not particularly limited and the PCR can be performed by a conventionally known method.

The sequence of the primer for PCR is not particularly limited as long as it can amplify the sequence to be detected. It can be designed suitably by a conventionally known method according to the target sequence. The region to be amplified may be, for example, only the sequence to be detected or it may be a region containing the sequence to be detected. The length of the primer is not particularly limited and can be set at a common length, for example, 10 to 30 mer. As described above, when the point mutation of the 758th base A (A to T) in the abl gene (Sequence No. 1) is to be detected, the primers consisting of the polynucleotides indicated below as specific examples can be used. The combination of those primers is not particularly limited. When the polynucleotide consisting of the base sequence of Sequence No. 9 (sense primer) is combined with the polynucleotide consisting of the base sequence of Sequence No. 10 (antisense primer), the resultant amplification product has a length of about 103 mer.

### (Primer set 1)

Sense primer Sequence No. 9
5'-ggagatggaacgcacggac-3' (GC content: 63.2%)

Antisense primer Sequence No. 10
5'-ggccaccgtcaggctg-3' (GC content: 75%)

### (Primer set 2)

Sense primer Sequence No. 11
5'-gacaagtgggagatggaacgc-3'

Antisense primer Sequence No. 12
5'-cacggccaccgtcagg-3'

Then prior to the hybridization step described below, the inhibitory polynucleotide is added to the sample containing the amplification product. The ratio of the inhibitory polynucleotide to be added is as mentioned above. As described above, the timing of addition is not limited to this. For example, the inhibitory polynucleotide can be added before or after or simultaneously with addition of the detection probe. Furthermore, the inhibitory polynucleotide may be added, for example, either before or after the gene amplification treatment but preferably before the gene amplification treatment because the treatment is simple and easy. As described above, in the case where the inhibitory polynucleotide is added before the gene amplification treatment, an additional phosphate group may have been added to the 3'-terminal thereof to prevent the inhibitory polynucleotide itself from elongating.

When the point mutation of the 758th base A (A to T) in the abl gene (Sequence No. 1) is to be detected, the inhibitory polynucleotide is, for example, a polynucleotide consisting of the base sequence of Sequence No. 5 containing no point mutation. Preferably, this inhibitory polynucleotide is used, for example, in combination with the detection probe consisting of the aforementioned base sequence of Sequence No. 4.

Inhibitory polynucleotide for mutation A758T Sequence No. 5
5'-cctccccgTactggcccccg-3' (GC content: 80%)

Subsequently, the resultant amplification product is dissociated and the single-stranded DNAs obtained by dissociation are hybridized with the detection probe and with the inhibitory polynucleotide.

The heating temperature employed in the dissociation step is not particularly limited as long as it is a temperature at which the amplification product can be dissociated, and is, for example, at least 85°C, preferably 85°C to 95°C. The heating time also is not particularly limited and is, for example, 1 second to 10 minutes, preferably 1 second to 5 minutes.

Hybridization of the single-stranded DNA obtained by dissociation with the detection probe and hybridization of the single-stranded DNA with the inhibitory polynucleotide can be accomplished by, for example, lowering the heating temperature employed in the dissociation step after the dissociation step. The temperature condition is, for example, 40°C or lower.

Volumes and concentrations of the respective compositions of the reaction solution obtained in the hybridization step are not particularly limited. Specifically, the concentration of DNA contained in the reaction solution is, for example, 0.01 to 1 µM, preferably 0.1 to 0.5 µM, the concentration of the detection probe is, for example, preferably in the range that meets the ratio of the detection probe to be added to the DNA, for example, 0.001 to 10 µM, preferably 0.001 to 1 µM, and the concentration of the inhibitory polynucleotide is, for example, 0.1 nM to 1 mM, preferably 0.1 nM to 100 µM.

In the present invention, addition of the inhibitory polynucleotide is important as described above, and, for example, concentrations of the DNA to be detected, the detection probe, and the inhibitory polynucleotide are not particularly limited. Specifically, in detection of a signal described below, for example, the concentration of the DNA to be detected in the reaction solution can be lowered when the detection sensitivity of an apparatus to be used is relatively higher, and preferably, the concentration of the DNA to be detected in the reaction solution is increased when the detection sensitivity of the apparatus to be used is relatively lower. Specifically, when a commercially available apparatus (trade name: Smart Cycler; manufactured by Cepheid) is used for the signal detection described below, it is preferable that in the reaction solution, for example, the concentration of the DNA to be detected be 5 to 1000 nM, the concentration of the detection probe be 50 to 1000 nM, and the concentration of the inhibitory polynucleotide be 5 nM to 100 µM, and it is more preferable that the concentration of the DNA to be detected be 10 to 500 nM, the concentration of the detection probe be 100 to 500 nM, and the concentration of the inhibitory polynucleotide be 10 nM to 50 µM.

Then the hybridization product formed of the single-stranded DNA and the labeled probe or the inhibitory polynucleotide is heated and thereby the signal variations caused by an increase in temperature are measured. For example, when Q-Probe is used, the probe hybridized to a single-stranded DNA shows weakened (or quenched) fluorescence and the probe released by dissociation emits fluorescence. Therefore, for example, it is only necessary to measure the increase in fluorescence intensity with an increase in temperature while the hybridization product whose fluorescence is being weakened (or quenched) is heated gradually.

The temperature range to be employed for the measurement of signal variations is not particularly limited. The starting temperature is, for example, room temperature (for instance, 10°C) to 85°C, preferably 25 to 70°C, and the end temperature is, for example, 40 to 105°C. The rate of temperature increase is not particularly limited and is, for example, 0.1 to 20°C/second, preferably 0.3 to 5°C/second.

Subsequently, the signal variations are analyzed to determine the Tm value. Specifically, the value at each temperature (-d (fluorescence intensity increment) / dt) is calculated from the fluorescence intensity obtained above, and the temperature at which the lowest value is obtained is determined as the Tm value. Furthermore, the temperature at which the highest fluorescence intensity increment per unit time (fluorescence intensity increment/t) is obtained also can be determined as the Tm value. In contrast to this case, the decrement of fluorescence intensity is to be measured when not a quenching probe but a probe that shows no signal by itself but shows a signal after hybridization is used as the detection probe.

The Tm value also can be calculated using, for example, conventionally known MELTCALC software (http://www.meltcalc.com/), or can be determined by the nearest neighbor method.

In the present invention, instead of the method of measuring the signal variations caused by an increase in temperature while the hybridization product is being heated as described above, for example, the signal variations caused during hybridization may be determined. That is, while the temperature of the sample containing the probe added thereto is decreased and thereby a hybridization product is formed, the signal variations caused by a decrease in temperature described above may be measured.

Specific examples are described below. In the case of using, as the detection probe, a labeled probe (for example, Q-Probe) that shows a signal by itself but shows no signal when hybridized, when the probe has been added to the sample, the detection probe emits fluorescence because it is in the dissociated form, but the fluorescence is weakened (or quenched) when it is hybridized by a decrease in temperature. Therefore, for example, with the temperature of the sample being decreased gradually, the decrease in fluorescence intensity with a decrease in temperature can be measured. On the other hand, in the case of using, as the detection probe, a labeled probe that shows no signal by itself but shows a signal when hybridized, when the probe has been added to the sample, the probe does not emit fluorescence because it is in the dissociated form but it starts emitting fluorescence when it is hybridized by a decrease in temperature. Therefore, for example, with the temperature of the sample being decreased gradually, the increase in fluorescence intensity with a decrease in temperature can be measured.

The detection probe kit of the present invention is a kit to be used in the method of detecting a mutation of the invention as described above and is characterized by including a detection probe consisting of a polynucleotide complementary to a sequence to be detected and an inhibitory polynucleotide complementary to a sequence not to be detected. In the present invention, the sequence to be detected is, as described above, a DNA to be detected in which a detection site has been mutated or a partial sequence thereof and contains the detection site that has been mutated. The sequence not to be detected is, as described above, the DNA not to be detected in which the detection site is unmutated or a partial sequence thereof and contains the detection site that is unmutated. As long as the detection probe kit of the present invention contains the probe and the inhibitory polynucleotide, other constituents are not limited.

The probe and the inhibitory polynucleotide are not limited and those similar to the aforementioned ones can be used and preferable combinations thereof also are as described above.

In the detection probe kit of the present invention, the probe and the inhibitory polynucleotide may be mixed into a single reagent or may be used as separate reagents independent from each other. In the former case, it is preferable that the probe and the inhibitory polynucleotide be mixed at the aforementioned ratio. In the latter case, they are used so that, for example, the ratios thereof in the reaction solution are in the range as described above. The detection probe kit of the present invention further may include a primer for amplifying a region containing the sequence complementary to the probe.

The data analysis method of the present invention is a method of analyzing data for determining the presence or absence of a DNA mutation and is characterized by including the following steps (a) and (b):
(a) a step of calculating a Tm value by analyzing signal variations obtained in the step (C) in the method of detecting a mutation of the present invention, and
(b) a step of determining the presence or absence of a DNA mutation from the Tm value calculated in the step of calculating.

The system of the present invention is a system for detecting the presence or absence of a DNA mutation in the sample and is characterized by including an input unit for inputting the signal variations obtained in step (C) in the method of detecting a mutation of the present invention, a calculation unit for calculating the Tm value from the signal variations inputted by the input unit, and a determination unit for determining the presence or absence of a DNA mutation based on the Tm value calculated by the calculation unit. The system of the present invention is, for example, a detector constructed of a computer system. The hardware structure of the system is not limited. For example, memory and input devices such as a keyboard and a mouse are connected to a controlling device CPU, to which, for example, a result output device and a display for displaying input data and results may be connected additionally. Each unit is only required to be a functional block that is implemented through execution of a predetermined program by the CPU of the computer. Therefore, for example, each constructive unit is not necessarily required to be mounted as hardware and may be in a network system.

The system of the present invention may have, for example, a unit for implementing step (B) and step (C) in the method of detecting a mutation of the present invention. The unit for implementing the aforementioned steps are, for example, a temperature control unit and a signal detection unit. Implementation of the temperature control unit allows, for example, formation of a double-stranded DNA by hybridization (hybridization product) and dissociation of a hybridization product. Implementation of the signal detection unit allows, for example, detection of the magnitude of signal variation caused by the formation of a hybridization product associated with a change in temperature and detection of the magnitude of signal variation caused by dissociation of a hybridization product. In this case, the system may include a recording unit for recording signal variations detected by the signal detection unit, in place of the input unit. Furthermore, the system of the present invention may be provided with a unit for outputting the presence or absence of a DNA mutation determined by the determination unit.

The program of the present invention is a computer program capable of executing the data analysis method of the present invention on a computer.

The electronic medium of the present invention is a computer-readable electronic medium (also referred to as a 'recording medium') in which the computer program of the present invention has been stored.

In the following, examples of the present invention are described together with comparative examples. The present invention is not limited by the following examples and comparative examples.

### [Example 1]

### Point mutation at 758th base in abl gene (A to T)

### (1) With an inhibitory polynucleotide added, the Tm analysis with respect to the point mutation at the 758th base (A to T) in the abl gene was performed.

Genomic DNA derived from a leukocyte cell line containing no mutation at the 758th base in the abl gene (Sequence No. 1) and genomic DNA derived from a leukocyte cell line containing a mutation at the 758th base in the abl gene (the 758th base is T in Sequence No. 1: abl tyrosinkinase A758T (=Y253F)) were prepared. Hereinafter, the former containing no mutation is referred to as 'wtDNA' and the latter containing a mutation as 'mtDNA'. These two were mixed at predetermined ratios (mtDNA : wtDNA = 100 : 0, 50 : 50, 10 : 90, 5 : 95, 3 : 97, 0 : 100). Then 10⁴ copy/test (1 µL) of the resultant mixture was added to 50 µL of the PCR reaction solution described below and thereby a PCR reaction was carried out. The final concentration of the inhibitory polynucleotide in the aforementioned PCR reaction solution was 200 nM and the final concentration of the detection probe was 50 nM. In the PCR reaction, using a thermal cycler, the PCR reaction solution was treated at 95°C for 60 seconds and then a cycle in which it was treated at 95°C for 10 seconds and at 60°C for 30 seconds was repeated for 50 cycles. Thereafter, it further was treated at 95°C for 1 second and at 40°C for 60 seconds. Subsequently, while the PCR reaction solution was heated from 40°C to 95°C at a rate of temperature increase of 1°C / 3 seconds, the change in fluorescence intensity with time was measured. The measurement wavelength was 585 to 700 nm. The ratio of the detection probe added was 0.1 times that of the PCR amplification product in molar ratio, and the ratio of the inhibitory polynucleotide added was 0.4 times that of the PCR amplification product in molar ratio and 4 times that of the probe in molar ratio. The ratio of the detection probe to the DNA to be detected (amplification product of the sequence to be detected) is as follows. These results are referred to as Example 1-1. In Comparative Example 1-1, fluorescence intensity was measured in the same manner except that 2 µL of distilled water, in place of 2 µL of the inhibitory polynucleotide, was added to the following PCR reaction solution.

**Table 1**

| Sample (mtDNA: wtDNA) | Ratio to DNA to be detected |
|---|---|
| 100:0 | 0.1 |
| 50: 50 | 0.2 |
| 10:90 | 1 |
| 5:95 | 2 |
| 3: 97 | 3.3 |
| 0:100 | - |

**Table 2 (PCR reaction solution: in µl)**

| | |
|---|---|
| Distilled water | 34.375 |
| 10 × gene Taq buffer* | 5 |
| 40% Glycerol | 3.125 |
| 2.5mM dNTPs | 4 |
| 100 µM sense primer | 0.5 |
| 100 µM antisense primer | 0.25 |
| 5 µM detection probe | 0.5 |
| 5 µM inhibitory polynucleotide | 2 |
| 5 U/µL Gene Taq FP* | 0.25 |
| Total | 50µL |

| | |
|---|---|
| * Trade name: Gene Taq Fp: manufactured by Nippon Gene (hereinafter, the same applies) | |

Sense primer Sequence No. 9
5'-ggagatggaacgcacggac-3'

Antisense primer Sequence No. 10
5'-ggccaccgtcaggctg-3'

Detection probe Sequence No. 4
5'-(TAMRA)-ccgAactggcccccgc-P-3'

Inhibitory polynucleotide Sequence No. 5
5'-cctccccgTactggcccccg-3'

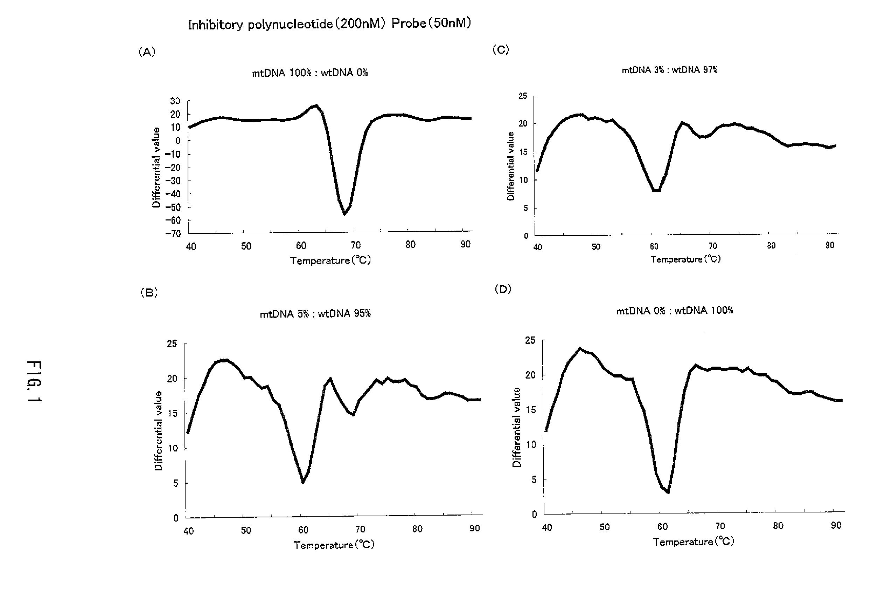

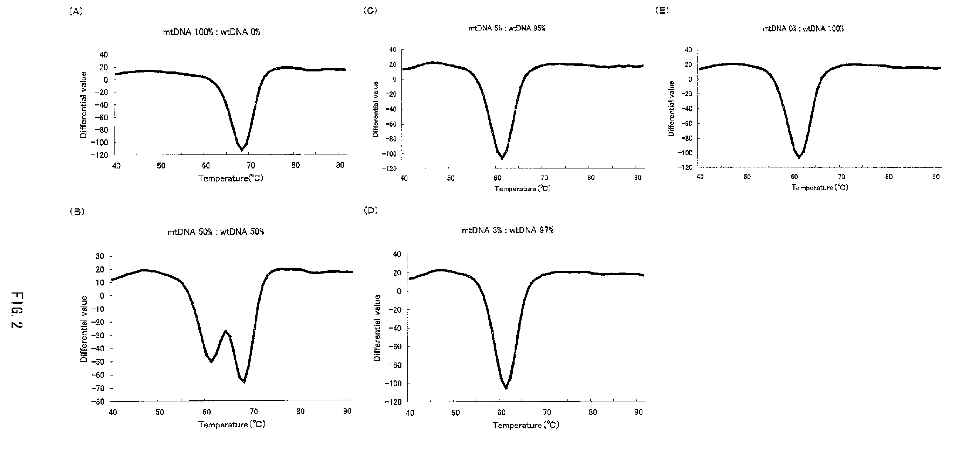

These results are shown in FIG. 1 and FIG. 2. Both the figures show graphs of Tm analysis indicating the change in fluorescence intensity with an increase in temperature. In the graphs, the differential value indicated on the vertical axis represents "-d (fluorescence intensity increment) / dt" (hereinafter, the same applies). FIG. 1 shows the result of Example 1-1 and FIG. 2 the result of Comparative Example 1-1. In FIG. 1, (A) shows the result of the sample in which 100% of the whole genome has point mutations, (B) the result of the sample in which 5% of the whole genome has point mutations, (C) the result of the sample in which 3% of the whole genome has point mutations, and (D) the result of the sample in which the whole genome has no point mutation. In FIG. 2, (A) shows the result of the sample in which 100% of the whole genome has point mutations, (B) the result of the sample in which 50% of the whole genome has point mutations, (C) the result of the sample in which 5% of the whole genome has point mutations, (D) the result of the sample in which 3% of the whole genome has point mutations, and (E) the result of the sample in which the whole genome has no point mutation.

The peak signal was detected at 69.5°C for mtDNA as shown in FIG. 1(A) and FIG. 2(A) and at 61.5°C for wtDNA as shown in FIG. 1(D) and FIG. 2(E). Each sample was evaluated with these being considered as the reference values. As a result, in Comparative Example 1-1 in which the inhibitory polynucleotide was not added, the signal of mtDNA was detected as shown in FIG. 2(B) when the sample contained 50% of mtDNA, but no signal of mtDNA was detected as shown in FIGs. 2(C) and (D) when the sample contained only a small amount (5%, 3%) of mtDNA. On the other hand, in Example 1-1 in which the inhibitory polynucleotide was added, the signal of mtDNA was detected as shown in FIGs. 1(B) and (C) even when the sample contained only a small amount (5%, 3%) of mtDNA. That is, addition of the inhibitory polynucleotide inhibited hybridization of the detection probe to wtDNA containing no point mutation, so that the amount of the detection probe to be bound to mtDNA was increased, which improved the detection sensitivity.

### (2) Furthermore, the detection probe was added at different ratios and Tm analysis was performed with respect to the point mutation at the 758th base in the abl gene (A to T).

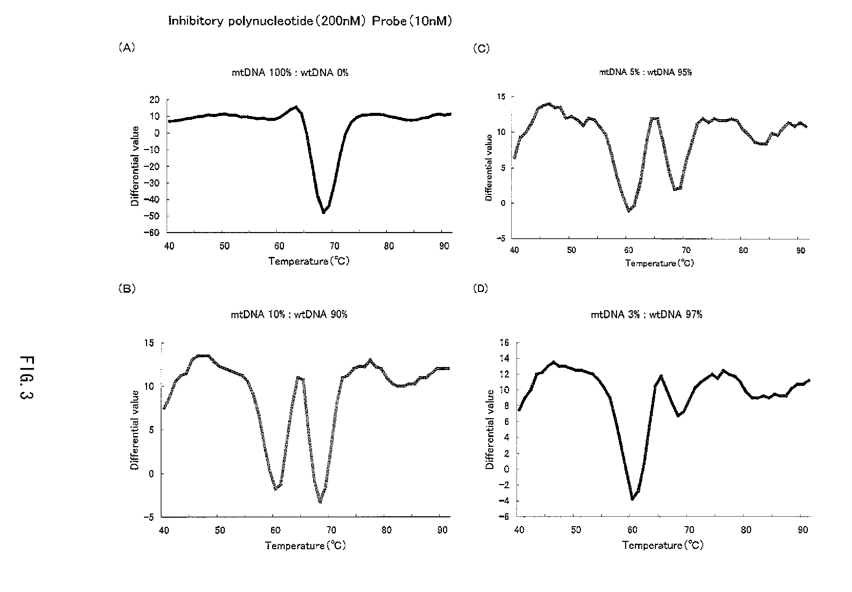

The Tm analysis was performed in the same manner as in Example 1 described above except that the amount of the detection probe to be added in the PCR reaction solution in Example 1 was changed from 0.5 µL to 0.1 µL, and the amount of distilled water to be added was changed from 34.375 µL to 34.775 µL, so that the final concentration of the detection probe was decreased to 1/5, 10 nM. The result is referred to as Example 1-2 and is shown in FIG. 3. In FIG. 3, (A) shows the result of the sample in which 100% of the whole genome has point mutations, (B) the result of the sample in which 10% of the whole genome has point mutations, (C) the result of the sample in which 5% of the whole genome has point mutations, and (D) the result of the sample in which 3% of the whole genome has point mutations.

As shown in FIG. 3, the peak of mtDNA was detected clearly. For example, as compared with the result of Example 1-1 in which the analysis was performed under the same conditions other than the amount of probe added, a further decrease in amount of the probe added from the amount thereof used in Example 1-1 (to 0.02 times the amount of the PCR amplification product, in molar ratio) further lowered the peak of wtDNA, which resulted in a higher relative peak of mtDNA, as shown in FIG. 3(C) versus FIG. 1(B) mentioned above and in FIG. 3(D) versus FIG. 1(C). This proved that adjustment of the amount of the probe to be added further improved the detection sensitivity.

### (3) The amount of the inhibitory polynucleotide to be added was increased and Tm analysis with respect to the point mutation at the 758th base in the abl gene (A to T) was performed.

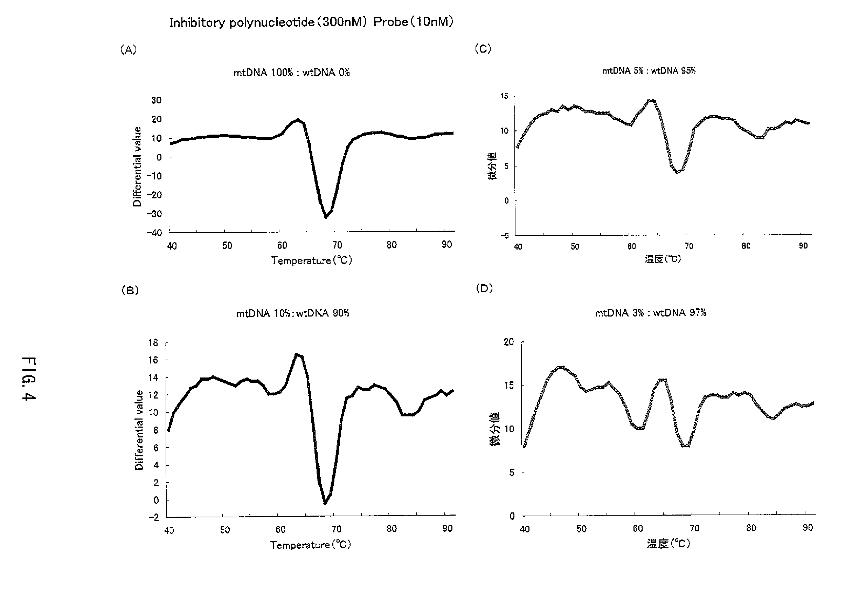

The Tm analysis was performed in the same manner as in Example 1-2 described in (2) above except that the addition amount of the inhibitory polynucleotide of the PCR reaction solution in Example 1 was changed from 2 µL to 3 µL, and the amount of distilled water to be added was changed from 34.375 µL to 33.775 µL. The final concentration of the inhibitory polynucleotide in the PCR reaction solution was 300 nM and the final concentration of the detection probe was 10 nM. The result is referred to as Example 1-3 and is shown in FIG. 4. In FIG. 4, (A) shows the result of the sample in which 100% of the whole genome has point mutations, (B) the result of the sample in which 10% of the whole genome has point mutations, (C) the result of the sample in which 5% of the whole genome has point mutations, and (D) the result of the sample in which 3% of the whole genome has point mutations.

As shown in FIG. 4, the peak of mtDNA was detected very clearly. Particularly, as compared with the result of Example 1-2 in which the analysis was performed under the same conditions other than the amount of the inhibitory polynucleotide added, an increase in amount of the inhibitory polynucleotide added from the amount thereof used in Example 1-2 (to 30 times the amount of the aforementioned probe, in molar ratio) further lowered the peak of wtDNA, which resulted in a higher relative peak of mtDNA, as shown in FIG. 4(B) versus FIG. 3(B) mentioned above, FIG. 4(C) versus FIG. 3(C), and FIG. 4(D) versus FIG. 3(D). This proved that adjustment of the amount of the inhibitory polynucleotide to be added further improved the detection sensitivity.

### [Example 2]

### Point mutation at 756th base in abl gene (G to C)

With an inhibitory polynucleotide added, the Tm analysis with respect to the point mutation at the 756th base (G to C) in the abl gene was performed.

A plasmid into which a normal abl gene sequence containing no mutation at the 756th base G of Sequence No. 1 had been inserted and a plasmid into which a mutated abl gene with the 756th base G mutated to C (abl tyrosinkinase G756C (= Q250E)) had been inserted were prepared. Hereinafter, the former containing no mutation is referred to as 'wtDNA', and the latter containing a mutation as 'mtDNA'. In the same manner as in Table 1 in Example 1 described above, these two were mixed at predetermined ratios (mtDNA : wtDNA= 0 : 100, 3 : 97, 100 : 0). Then 10⁴ copy/test (1 µL) of the resultant mixture was added to 49 µL of the PCR reaction solution described below and thereby a PCR reaction was carried out. The final concentration of the inhibitory polynucleotide in the PCR reaction solution was 300 nM and the final concentration of the detection probe was 50 nM. In the PCR reaction, using a thermal cycler, the PCR reaction solution was treated at 95°C for 60 seconds and then a cycle in which it was treated at 95°C for 1 second and at 58°C for 30 seconds was repeated for 50 cycles. Thereafter, it further was treated at 95°C for 1 second and at 40°C for 60 seconds. Subsequently, while the PCR reaction solution was heated from 40°C to 95°C at a rate of temperature increase of 1°C / 3 seconds, the change in fluorescence intensity with time was measured. The measurement wavelength was 585 to 700 nm. The ratio of the detection probe added was 0.05 times that of the PCR amplification product in molar ratio, the ratio of the inhibitory polynucleotide added was 0.3 times that of the PCR amplification product in molar ratio and 6 times that of the probe in molar ratio. In Comparative Example 2, fluorescence intensity was measured in the same manner except that 3 µL of distilled water, in place of 3 µL of the inhibitory polynucleotide, was added to the following PCR reaction solution.

**Table 3 (PCR reaction solution: in µl)**

| | |
|---|---|
| Distilled water | 21.75 |
| 10 × gene Taq buffer* | 5 |
| 40% Glycerol | 12.5 |
| 2.5 mM dNTPs | 4 |
| 100 mM MgCl₂ | 0.5 |
| 100 nM sense primer | 1 |
| 100 µM antisense primer | 0.5 |
| 5 µM detection probe | 0.5 |
| 5 µM inhibitory polynucleotide | 3 |
| 5 U/µL Gene Taq FP* | 0.25 |
| Total | 49 µL |

Sense primer Sequence No. 13
5'-gacaagtgggagatggaacgc-3'

Antisense primer Sequence No. 14
5'-cacggccaccgtcagg-3'

Detection probe Sequence No. 2
5'-(BODIPY FL)-ccgtaGtggcccccgc-P-3'

Inhibitory polynucleotide Sequence No. 3
5'-ccgtaCtggcccccgc-P-3'

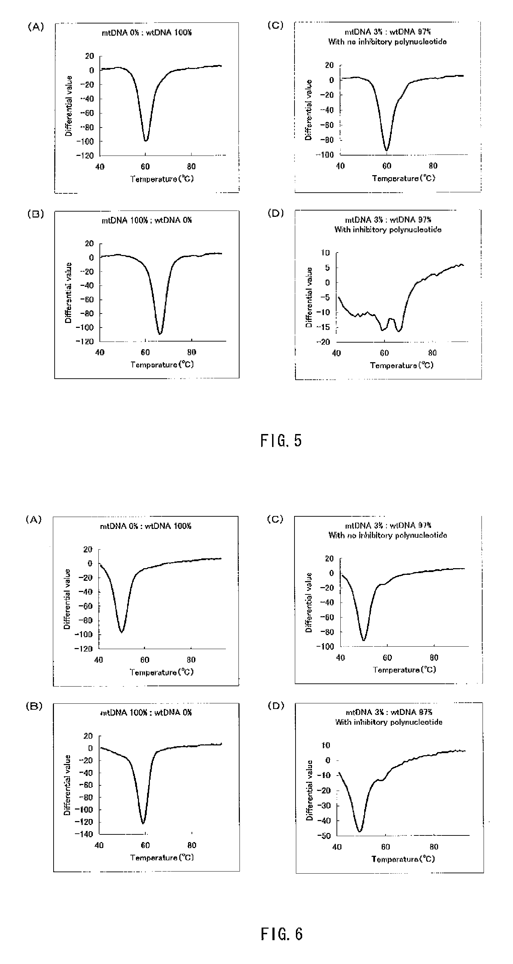

These results are shown in FIG. 5. FIG. 5 shows a graph of Tm analysis indicating the change in fluorescence intensity with an increase in temperature. In FIG. 5, (A) shows the result of a sample in which the whole plasmid has no point mutation, (B) the result of a sample in which 100% of the whole plasmid has point mutations, and (C) as well as (D) the results of samples in which 3% of the whole plasmid has point mutations. The aforementioned (C) shows the result of Comparative Example 2 and the aforementioned (D) the result of Example 2.

The peak signal was detected at 61.0°C for wtDNA as shown in FIG. 5(A) and at 67.0°C for mtDNA as shown in FIG. 5(B). The sample in which 3% of the whole plasmid had point mutations was evaluated with these being considered as the reference values. As a result, in Comparative Example 2 in which the inhibitory polynucleotide was not added, no signal of mtDNA was detected as shown in FIG. 5(C). On the other hand, in Example 2 in which the inhibitory polynucleotide was added, both the signal of wtDNA and the signal of mtDNA were detected as shown in FIG. 5(D), even when the sample contained only a small amount of mtDNA.

### [Example 3]

### Point mutation at 763rd base in abl gene (G to A)

With an inhibitory polynucleotide added, the Tm analysis with respect to the point mutation at the 763rd base (G to A) in the abl gene was performed.

A plasmid into which a normal abl gene sequence containing no mutation at the 763rd base G of Sequence No. 1 had been inserted and a plasmid into which a mutated abl gene with the 763rd base G mutated to A (abl tyrosinkinase G763A (= E255K)) had been inserted were prepared. Hereinafter, the former containing no mutation is referred to as 'wtDNA', and the latter containing a mutation as 'mtDNA'. In the same manner as in Table 1 in Example 1 described above, these two were mixed at predetermined ratios (mtDNA : mtDNA= 0 : 100, 3 : 97, 100 : 0). Then, 10⁴ copy/test (1 µL) of the resultant mixture was added to 49 µL of the PCR reaction solution described below and thereby a PCR reaction was carried out. The final concentration of the inhibitory polynucleotide in the PCR reaction solution was 500 nM and the final concentration of the detection probe was 50 nM. The PCR reaction was performed in the same manner as in Example 2 and the change in fluorescence intensity with time was measured. The measurement wavelength was 585 to 700 nm. The ratio of the detection probe added was 0.05 times that of the PCR amplification product in molar ratio, and the ratio of the inhibitory polynucleotide added was 0.5 times that of the PCR amplification product in molar ratio and 10 times that of the probe in molar ratio. In Comparative Example 3, the fluorescence intensity was measured in the same manner except that 5 µL of distilled water, in place of 5 µL of the inhibitory polynucleotide, was added to the following PCR reaction solution.

**Table 4 (PCR reaction solution: in µl)**

| | |
|---|---|
| Distilled water | 23.375 |
| 10 × gene Taq buffer* | 5 |
| 40% Glycerol | 9.375 |
| 2.5 mM dNTPs | 4 |
| 100 µM sense primer | 0.5 |
| 100 µM antisense primer | 1 |
| 5 µM detection probe | 0.5 |
| 5 µM inhibitory polynucleotide | 5 |
| 5 U/µL Gene Taq FP* | 0.25 |
| Total | 49 µL |

Sense primer Sequence No. 15
5'-gacaagtgggagatggaacgc-3'

Antisense primer Sequence No. 16
5'-cacggccaccgtcagg-3'

Detection probe Sequence No. 7
5'-(BODIPY FL)-ccagtacgggAaggtgt-P-3'

Inhibitory polynucleotide Sequence No. 8
5'-ccagtacgggGaggtgt-P-3'

These results are shown in FIG. 6. FIG. 6 shows a graph of Tm analysis indicating the change in fluorescence intensity with an increase in temperature. In FIG. 6, (A) shows the result of a sample in which the whole plasmid has no point mutation, (B) the result of a sample in which 100% of the whole plasmid has point mutations, and (C) as well as (D) the results of samples in which 3% of the whole plasmid has point mutations. The aforementioned (C) shows the result of Comparative Example 3 and the aforementioned (D) the result of Example 3.

The peak signal was detected at 50.0°C for wtDNA as shown in FIG. 6(A) and at 59.0°C for mtDNA as shown in FIG. 6(B). The sample in which 3% of the whole plasmid had point mutations was evaluated with these being considered as the reference values. As a result, in Comparative Example 3 in which the inhibitory polynucleotide was not added, no signal of mtDNA was detected as shown in FIG. 6(C). On the other hand, in Example 3 in which the inhibitory polynucleotide was added, both the signal of wtDNA and the signal of mtDNA were detected as shown in FIG. 6(D), even when the sample contained only a small amount of mtDNA.

### [Example 4]

### Point mutation at 758th base in abl gene (A to T)

With an inhibitory polynucleotide added, the Tm analysis with respect to the point mutation at the 758th base (A to T) in the abl gene was performed.

A plasmid into which a normal abl gene sequence containing no mutation at the 758th base A of Sequence No. 1 had been inserted and a plasmid into which a mutated abl gene with the 758th base A mutated to T (abl tyrosinkinase A758T (= Y253F)) had been inserted were prepared. Hereinafter, the former containing no mutation is referred to as 'wtDNA' and the latter containing a mutation as 'mtDNA'. In the same manner as in Table 1 in Example 1 described above, these two were mixed at predetermined ratios (mtDNA : wtDNA = 0 : 100, 3 : 97, 1.00 : 0). Then 10⁴ copy/test (1 µL) of the resultant mixture was added to 49 µL of the PCR reaction solution described below and thereby a PCR reaction was carried out. The final concentration of the inhibitory polynucleotide in the PCR reaction solution was 500 nM and the final concentration of the detection probe was 50 nM. The PCR reaction was performed in the same manner as in Example 2 and the change in fluorescence intensity with time was measured. The measurement wavelength was 585 to 700 nm. The ratio of the detection probe added was 0.05 times that of the aforementioned PCR amplification product in molar ratio, and the ratio of the inhibitory polynucleotide added was 0.5 times that of the PCR amplification product in molar ratio and 10 times that of the probe in molar ratio. In Comparative Example 4, fluorescence intensity was measured in the same manner except that 5 µL of distilled water, in place of 5 µL of the inhibitory polynucleotide, was added to the following PCR reaction solution.

**Table 5 (PCR reaction solution: in µl)**

| | |
|---|---|
| Distilled water | 20.25 |
| 10 × gene Taq buffer* | 5 |
| 40% Glycerol | 12.5 |
| 2.5 mM dNTPs | 4 |
| 100 µM sense primer | 1 |
| 100 µM antisense primer | 0.5 |
| 5 µM detection probe | 0.5 |
| 5 µM inhibitory polynucleotide | 5 |
| 5 U/µL Gene Taq Fop* | 0.25 |
| Total | 49 µL |

Sense primer Sequence No. 11
5'-gacaagtgggagatggaacgc-3'

Antisense primer Sequence No. 12
5'-cacggccaccgtcagg-3'

Detection probe Sequence No. 4
5'-(TAMRA)-ccgAactggcccccgc-P-3'

Inhibitory polynucleotide Sequence No. 6
5'-ccgTactggcccccgc-P-3'

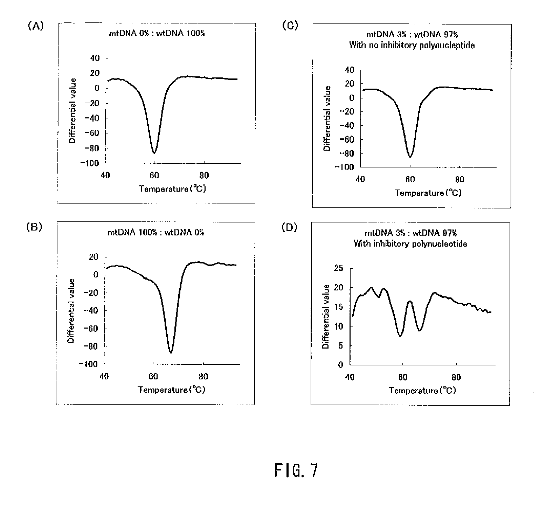

These results are shown in FIG. 7. FIG. 7 shows a graph of Tm analysis indicating the change in fluorescence intensity with an increase in temperature. In FIG. 7, (A) shows the result of a sample in which the whole plasmid has no point mutation, (B) the result of a sample in which 100% of the whole plasmid has point mutations, and (C) as well as (D) the results of samples in which 3% of the whole plasmid has point mutations. The aforementioned (C) shows the result of Comparative Example 4 and the aforementioned (D) the result of Example 4.

The peak signal was detected at 60.0°C for wtDNA as shown in FIG. 7(A) and at 67.0°C for mtDNA as shown in FIG. 7(B). The sample in which 3% of the whole plasmid had point mutations was evaluated with these being considered as the reference values. As a result, in Comparative Example 4 in which the inhibitory polynucleotide was not added, no signal of mtDNA was detected as shown in FIG. 7(C). On the other hand, in Example 4 in which the inhibitory polynucleotide was added, both the signal of wtDNA and the signal of mtDNA were detected as shown in FIG. 7(D), even when the sample contained only a small amount of mtDNA.

### Industrial Applicability

As mentioned above, the method of detecting a mutation of the present invention enables, by adding the aforementioned inhibitory polynucleotide, detection of a point mutation at an excellent sensitivity even in a sample that contains both a DNA to be detected containing a point mutation and a DNA not to be detected containing no point mutation, such as a leukocyte sample from a leukemia patient. Furthermore, the method can identify the type of the mutation and therefore it also is excellent in specificity. Accordingly, the method is useful particularly for detection of a point mutation in leukemia patients, and is very useful in the field of medical care because, for example, it makes it possible to analyze at the gene level whether or not a therapeutic drug for leukemia is suitable for individual patients.
## Claims

1. A method of detecting a mutation of a DNA contained in a sample,
wherein the sample contains a DNA to be detected in which a detection site has been mutated and a DNA not to be detected in which the detection site is unmutated, and
the method comprises the following steps (A) to (E):
(A) adding a detection probe consisting of a polynucleotide complementary to a sequence to be detected and a polynucleotide complementary to a sequence not to be detected, to the sample containing the DNA,
wherein the sequence to be detected is the DNA to be detected or a partial sequence thereof and contains the detection site that has been mutated, and
the sequence not to be detected is the DNA not to be detected or a partial sequence thereof and contains the detection site that is unmutated,
(B) hybridizing the detection probe to the DNA,
(C) measuring a signal variation associated with a change in temperature with respect to a hybridization product between the DNA and the detection probe,
(D) determining a Tm value based on analysis of the signal variation, and
(E) determining the presence or absence of a mutation at the site to be detected, based on the Tm value.

2. The method of detecting a mutation according to claim 1, wherein the detection probe is a probe labeled with a labeling substance.

3. The method of detecting a mutation according to claim 2, wherein the labeled probe is a labeled probe that shows a signal by itself but shows no signal after hybridization or a labeled probe that shows no signal by itself but shows a signal after hybridization.

4. The method of detecting a mutation according to claim 2, wherein the labeling substance is a fluorochrome, and the labeled probe is a probe that emits fluorescence by itself and emits weakened fluorescence after hybridization.

5. The method of detecting a mutation according to claim 2, wherein the labeled probe is a probe in which a base of a 3'-terminal thereof is cytosine and the 3'-terminal has been labeled.

6. The method of detecting a mutation according to claim 2, wherein the labeled probe is a probe in which a base of a 5'-terminal thereof is cytosine and the 3'-terminal has been labeled.

7. The method of detecting a mutation according to claim 1, wherein a detection probe complementary to the sequence to be detected and a polynucleotide complementary to the sequence not to be detected are identical in sequence to each other except for a site to be paired with the detection site.

8. The method of detecting a mutation according to claim 1, wherein a ratio of the polynucleotide to be added is 0.1 to 100 times that of the detection probe in molar ratio, with the polynucleotide being complementary to the sequence not to be detected.

9. The method of detecting a mutation according to claim 1, wherein a ratio of the detection probe to be added is 1 or lower times that of the DNA in molar ratio.

10. The method of detecting a mutation according to claim 1, wherein the DNA is a DNA derived from a leukocyte.

11. The method of detecting a mutation according to claim 1, wherein the mutation is a mutation of an abl gene.

12. The method of detecting a mutation according to claim 1, wherein the mutation is a mutation of the 756th base G to C in a base sequence of Sequence No. 1.

13. The method of detecting a mutation according to claim 12, wherein the detection probe is a probe consisting of a base sequence of Sequence No. 2, and the polynucleotide complementary to the sequence not to be detected is a polynucleotide consisting of a base sequence of Sequence No. 3.

14. The method of detecting a mutation according to claim 1, wherein the mutation is a mutation of the 758th base A to T in a base sequence of Sequence No. 1.

15. The method of detecting a mutation according to claim 14, wherein the detection probe is a probe consisting of a base sequence of Sequence No. 4, and the polynucleotide complementary to the sequence not to be detected is a polynucleotide consisting of a base sequence of at least one of Sequence No. 5 and Sequence No. 6.

16. The method of detecting a mutation according to claim 1, wherein the mutation is a mutation of the 763rd base G to A in a base sequence of Sequence No. 1.

17. The method of detecting a mutation according to claim 16, wherein the detection probe is a probe consisting of a base sequence of Sequence No. 7, and the polynucleotide complementary to the sequence not to be detected is a polynucleotide consisting of a base sequence of Sequence No. 8.

18. The method of detecting a mutation according to claim 1, wherein the DNA contained in the sample is a double-stranded DNA, the method comprises dissociating the double-stranded DNA contained in the sample by heating before step (B).

19. The method of detecting a mutation according to claim 1, wherein the DNA is an amplification product obtained through amplification carried out by a gene amplification method, using, as a template, a DNA derived from the sample.

20. The method of detecting a mutation according to claim 1, wherein the DNA is an amplification product obtained through amplification carried out by a gene amplification method, using, as a template, a cDNA produced through a reverse transcription reaction from an RNA derived from the sample.

21. The method of detecting a mutation according to claim 1, wherein in step (C), the hybridization product between the DNA and the detection probe is heated and thereby the signal variation associated with an increase in temperature is measured.

22. The method of detecting a mutation according to claim 1, being a method in which step (B) and step (C) are carried out concurrently,
wherein a decrease in temperature of the sample allows the hybridization product to be formed, and thereby the signal variation associated with the decrease in temperature is measured.

23. A detection probe kit used in a method of detecting a mutation according to claim 1,
wherein the detection probe kit comprises a detection probe consisting of a polynucleotide complementary to a sequence to be detected and a polynucleotide complementary to a sequence not to be detected,
the sequence to be detected is a DNA to be detected in which a detection site has been mutated or a partial sequence thereof and contains the detection site that has been mutated, and
the sequence not to be detected is the DNA not to be detected in which the detection site is unmutated or a partial sequence thereof and contains the detection site that is unmutated.

24. The detection probe kit according to claim 23, wherein the detection probe is a probe labeled with a labeling substance.

25. The detection probe kit according to claim 23, wherein the detection probe complementary to the sequence to be detected and the polynucleotide complementary to the sequence not to be detected are identical in sequence to each other except for a site to be paired with the detection site.

26. The detection probe kit according to claim 23, wherein the mutation is a mutation of an abl gene.

27. The detection probe kit according to claim 23, wherein the mutation is a mutation of the 756th base G to A in a base sequence of Sequence No. 1, the detection probe is a probe consisting of a base sequence of Sequence No. 2, and the polynucleotide complementary to the sequence not to be detected is a polynucleotide consisting of a base sequence of Sequence No. 3.

28. The detection probe kit according to claim 23, wherein the mutation is a mutation of the 758th base A to T in a base sequence of Sequence No. 1, the detection probe is a probe consisting of a base sequence of Sequence No. 4, and the polynucleotide complementary to the sequence not to be detected is a polynucleotide consisting of a base sequence of at least one of Sequence No. 5 and Sequence No. 6.

29. The detection probe kit according to claim 23, wherein the mutation is a mutation of the 763rd base G to A in a base sequence of Sequence No. 1, the detection probe is a probe consisting of a base sequence of Sequence No. 7, and the polynucleotide complementary to the sequence not to be detected is a polynucleotide consisting of a base sequence of Sequence No. 8.

30. A method of analyzing data for determining the presence or absence of a DNA mutation,
wherein the method comprises steps (a) and (b):
(a) calculating a Tm value from a signal variation obtained in step (C) in a method of detecting a mutation according to claim 1, and
(b) determining the presence or absence of a DNA mutation from the Tm value calculated in the calculation step.

31. A system for detecting the presence or absence of a DNA mutation in a sample,
wherein the system comprises:
an input unit for inputting a signal variation obtained in step (C) in a method of detecting a mutation according to claim 1,
a calculation unit for calculating a Tm value from the signal variation inputted by the input unit, and
a determination unit for determining the presence or absence of a DNA mutation based on the Tm value calculated by the calculation unit.

32. A computer program capable of executing a method of analyzing data according to claim 30 on a computer.

33. An electronic medium in which a computer program according to claim 32 has been stored.
